# EUROPEAN PATENT APPLICATION

(11) **EP 3 216 393 A1**
(43) Date of publication of application: **13.09.2017**
(21) Application number: 17156094.9
(22) Date of filing: 14.02.2017
(51) Int. Cl.: A61B 5/053, A61B 5/00

(54) **A GLOVE PROVIDED WITH ELECTRODES FOR A CHECK OF THE PROSTATE**

(30) Priority: 07.03.2016 IT UA20161403
(71) Applicant: Akern S.r.L., 50065 Pontassieve (FI) (IT)
(72) Inventor: TALLURI, Antonio, 50012, Bagno a Ripoli (FI) (IT); DELORENZO, Antonino, 87020 Acquappesa (CS) (IT); DI RENZO, Laura, 00183 Roma (RM) (IT)
(74) Representative: Emmi, Mario

(57) **Abstract**

The present invention concerns a device (1) for the check of the prostate.

The device is a glove (3) comprising a first hole for the insertion of the hand inside it.

It comprises further at least a second hole (50; 70) such that, as a conductive receiving element **(Er1, Er2)** is arranged inside the glove in correspondence of said second hole (50; 70), it results at least in part not covered by the glove in direct contact with the outside to allow the implementation of a measurement according to the tetrapolar technique.

## Description

### Technical field

The present invention refers to the medical technical field.

In particular, the invention refers to a particularly useful glove for the diagnosis and the check of the prostate.

### Background art

The prostate check is very important both in terms of prevention and for diagnosis of various diseases.

The prostate, or prostatic gland, is the male organ part of the genital apparatus. Its main function is to produce and emit seminal fluid, one of the component of sperm, containing necessary elements for feeding and conducting spermatozoa. The male prostate can be palpated through rectal examination, being positioned approximately 5 cm forward the rectum.

Generally, the prostate examination requires the full bladder and the doctor may insert a probe to check its integrity or palpate it through a finger.

The examination made by palpation is always subjective, as it depends obviously on the ability and experience of the doctor to understand if everything is in order or not.

The examination made by a probe, for example ultrasound, is a little more certain, even if, also for this case, images have to be interpreted correctly and, besides, all parts of clinical interest have to be focused carefully. In addition, initial diseases are not always visible.

In medical field, medical devices basing its operation on the well-known tetra-polar technique are known. Such a technique foresees the injection of a current, generally through two electrodes and the measurement of a related voltage drop and then drawing on bioelectrical impedance data to interpret various physiological state of the patient, for example the measurement of the hydration condition, fat and lean body mass or even mouth lesions, for example as described in WO2015/189727.

The publication US6567990 in the name of Spitznagle is known for the purpose of rectal or vaginal inspections. It is specific of the field of checking the correct pelvic or rectal muscular activity and describes a glove equipped with two integrated electrodes arranged on the same finger of the glove. Both are connected, through a specific wiring to a suitable device for detecting electrical activity. Moreover, a covering is foreseen only for the sole finger with electrodes, in order to guarantee their better stability. For that purpose, the covering foresees holes in correspondence of the electrodes position, in such a manner that they result exposed towards the outside. When it is necessary to make a rectal inspection to check the correct muscular function, the operator puts on the glove and the covering on the finger. Then he proceeds with the insertion of the finger inside the rectum to bring the two electrodes in contact with the insides of the canal, in such a manner that the electric muscular activity (electromyography) can be detected and, on the basis of it, to determine a correct function or a potential disease. In fact, the muscular activity results in electric current which is detected by the two electrodes defining a circuit with the insides.

However, such a proposed solution is specific for determining muscular activity, therefore functional tests, and it is not able to diagnose prostatic tissues lesions, even of the tumorous type. In fact, the two electrodes are in fixed positions in correspondence of the sole finger to be inserted inside the rectum and two electrodes inside the rectum imply the impossibility to detect correctly the bioelectrical impedance data of the prostate. In fact, the area under examination saturated by current, is wider and therefore at least a receiving electrode has to be placed outside the rectum and distanced from it. Moreover, such a document does not describe and does not foresee a current injection according to the tetra-polar technique, but, on the contrary, the described system limits itself to detect suitable endogenous micro-currents to measure a muscular activity and therefore to detect a potential current produced by the muscles themselves.

Another prior art, specifically concerning the field of tetra-polar technique, foresees the use of a "portable" system to take bioelectrical impedance measurements. It is described in the publication EP2454991 and deals with a support wearable on a hand and equipped with grabbing pincers on which two electrodes and further two electrodes with relative processor for bioelectrical impedance measurements are placed. Pincers are suitable for grabbing the foot, by bringing the two electrodes in contact with skin to take the measurement.

Although the instrument is suitable for a multiplicity of measurement, it is obvious that its structure is not suitable for a measurement of the rectum. Moreover, the system is complex and integrates four electrodes in its structure, thus limiting again the possibility to place electrodes freely and monitoring the area to be saturated with current. Therefore, the operator, once worn the glove, is compelled to insert a finger with a sole electrode into the rectum and to bring contextually all other electrodes in contact with specific points of the body in close proximity of the rectum: the whole operation results practically impossible.

### Disclosure of the invention

It is therefore the aim of the present invention to provide an innovative glove, a receiving conductive element and in particular, an assembly which allow to solve said technical inconveniences.

In particular, the aim of the present invention is to provide a glove, as well as an assembly, which allow to inspect the prostate rapidly and simply, thus providing at the same time objective classification data through bioelectrical impedance parameters that can be interpreted potentially as health conditions of the prostate itself.

These and other aims are therefore obtained through a glove (3) for sanitary use for the check of the prostate, according to claim 1.

Such a glove for sanitary use, according to the invention, reproduces, obviously, the form of a hand, in such a manner so as to being worn normally.

According to the invention, it is provided exclusively with only one receiving electrode (**Er1, Er2**) arranged in correspondence of a finger of the glove and at least in part not covered by the material of the glove and thus resulting in direct contact with the outside.

In this way, all above-mentioned technical inconveniences are easily solved.

It is enough to apply externally normal electrodes, according to the tetra-polar technique, thus two injecting ones connected to the generator and a receiving one. The fourth receiving one is that included in the glove. The receiving electrodes are connected to the voltmeter and then the current injection is initiated. The operator can insert the finger inside the rectum and make the inspection.

Preferably, the other receiving electrode is arranged in proximity of testicles as per normal position during the palpation.

After the current injection, the receiving electrodes allow to trace back to the potential drop or phase variation in the prostatic area and therefore it is possible to check various bioelectrical impedance parameters for a precise diagnosis.

Contrary to the cited prior art, such a glove allows such a measurement according to the tetra-polar technique. In fact, contrary to US6567990 in the name of Spitznagle, it is possible to apply the second electrode outside the anus in the most suitable point, thus taking a tetra-polar measurement not obtainable de facto with the described US6567990 wherein the two electrodes are on the same finger in close position.

Equally, contrary to the solution proposed in EP2454991, the proposed solution is flexible in use, as, in this way, all the remaining electrodes, in particular the injecting and the receiving ones, are independent and therefore they can be placed in the most suitable positions.

As alternative, the above-mentioned solution may foresee two different electrodes in the glove but on two different fingers.

In particular, a solution wherein the glove includes only two receiving electrodes (**Er1, Er2**) may be foreseen, said electrodes being arranged one in correspondence of a finger of the glove and the other one in correspondence of another finger of the glove, different from the previous one, said two receiving electrodes resulting at least in part not covered by the material of the glove and thus resulting in direct contact with the outside.

In this way, for example arranging an electrode on the finger and the other one on the thumb, the operator can make the check by inserting a finger into the anus (preferably the index finger) and the other one (preferably the thumb) outside the anus in proximity of testicles.

Once again, such an alternative solution solves the problems of the above-mentioned prior art.

In particular, even if the glove according to the invention foresees now two electrodes, those are placed on two different and separated fingers, such that the operator can insert the finger with an electrode into the rectum and bring the other finger with the other electrode in contact with the external part, for example in proximity of testicles. As already mentioned, this solution would not be practicable with a solution describing two electrodes on the same finger.

Equally, the glove described in such an alternative embodiment is cooperating with separated injecting electrodes, thus allowing to saturate correctly the areas of interest under examination. This would not be practicable with the glove in EP2454991 which includes even four electrodes and the processor.

### Brief description of drawings

Further features and advantages of the present device 1, according to the invention, will result to be clearer with the description that follows of some embodiments, made to illustrate but not to limit, with reference to the attached drawings, wherein:
- Figure 1 shows a view of a glove according to the present invention and provided with two electrodes (Er1, Er2) to measure a potential difference drop according to the tetra-polar technique;
- Figures 2 and 3 show a working diagram of the present glove;

- Figure 4 shows the processing of measurements by a processor, as per prior art, in order to trace back to one or more bioelectrical impedance parameters;
- Figure 5 shows a particularly simple variation wherein the glove is provided with only one electrode;
- Figure 6 and figure 7 show constructional details related to the invention;
- Figure 8 and figure 9 show a pattern of diagrams that can be plotted on display and which delimit an area of normality, so that during a test may or may not occur that the subject under examination remains within that area of normality.

### Description of some preferred embodiments

Gloves for sanitary use, as it is well known, have good characteristics of sensibility, resistance against rips, impermeability, etc. For that purpose, they can be made of various materials, such as for example latex of natural rubber or of the synthetic type as micro-fabric of polychloroprene. These are only some of the examples, as the realization of gloves for sanitary use is itself very well known: such a technology will not be described in details further, also because it is not the specific object of the present invention.

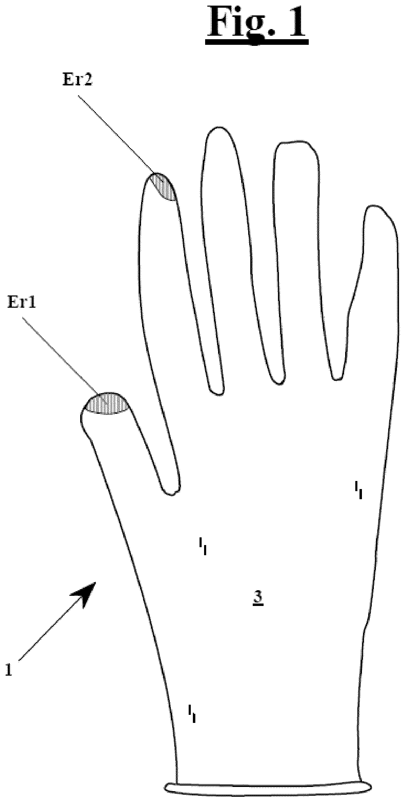

Figure 1 shows a glove for sanitary use according to a first embodiment of the present invention.

Regardless of the type, material and size of the glove for sanitary use, according to the invention, the glove is provided with a couple of receiving electrodes (Er1, Er2) for allowing the measurement of a potential difference drop, following the current injection, according to the tetra-polar technique.

Therefore, figure 1 shows the classic glove for sanitary use, having a body 3 shaping the form of the hand and a hole for inserting the hand inside it, according to a traditional use.

According to the invention, now, the glove includes such electrodes (Er1, Er2). The electrode Er1 can for example be arranged at the apex of the thumb while the electrode Er2 can be placed preferably at the apex of the index finger.

The position and the choice of the finger of the glove where to place electrodes is not necessarily those represented in figure 1 and, depending on uses of such a glove, other positions might be chosen, for example also in correspondence of other fingers and the use of more electrodes (not necessarily two but also more than two).

However, in case of checks of the prostate, the arrangement of figure 1 is a possible valid solution, considering that the check occurs traditionally through the use of the thumb and the finger to palpate the areas of interest.

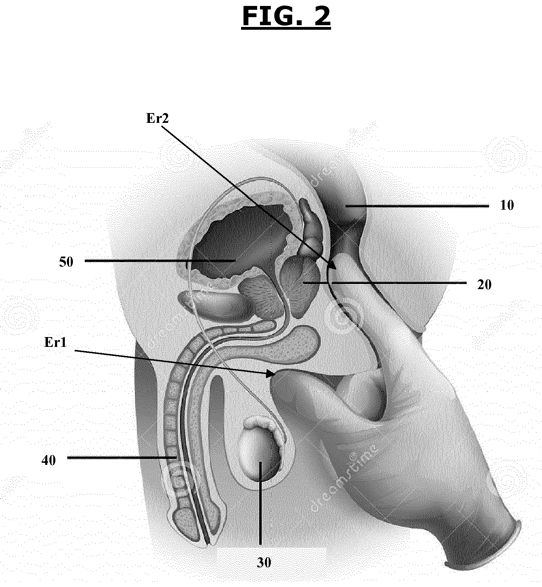

For this purpose, figure 2 shows and example of use.

Figure 2 shows clearly how the check takes place. The figure shows the rectum 10, the area of testicles 30 and of the penis 40. Then number 20 in figure shows the prostate being palpated during the check and the bladder 50.

The check, as per prior art, takes place through palpation, by inserting the index finger into the anus and positioning the thumb behind testicles. In this way, through the palpation, the doctor feels if the prostate has swellings and/or anomalies.

According to the invention, the glove foresees now the two receiving electrodes Er1 and Er2 indicated in their positioning also in figure 2.

At this point, according to the tetra-polar technique, it is enough to inject a constant current in such a manner so as to measure a related potential difference drop by means of a voltmeter (V) connected to the receiving electrodes and from here, with well-known formulas, trace back to bioelectrical impedance parameters, such as resistance, reactance and phase.

The tetra-polar technique has been known for a long time and it is not a specific object of the present invention but, for completeness, it is here briefly resumed.

Such a technique foresees the injection of a current by means of injecting electrodes. Then a generator is connected to such electrodes and generates the current passing through the body portion under examination.

The current is of the alternating type at one or more frequencies (for example 5Khz and 10khz).

It is generally foreseen also a feed-back operating principle (well-known by itself) which allows to maintain the constant supply of current (for example, a constant current of 400 microamperes, obviously harmless for human body).

The current saturating the part under examination is felt by the receiving electrodes (Er1, Er2) that allow to measure the consequent voltage drop, such electrodes being connected physically to a Voltmeter (V).

Then a processor is able to trace back to various measurements, such as resistance, reactance and phase, through knowledge of the voltage drop, as per prior art. Then such bioelectrical impedance parameters are usable in multiple ways.

As alternative to the voltmeter, or in combination with it, systems for detecting the phase can be used to determine a phase variation.

In a well-known case, which is not the object of the present invention, for example such bioelectrical impedance parameters are useful to trace back to the measurement of the hydration condition or of the fat mass.

According to the present invention, such parameters are instead used to understand if the prostate may be affected by potential anomalies.

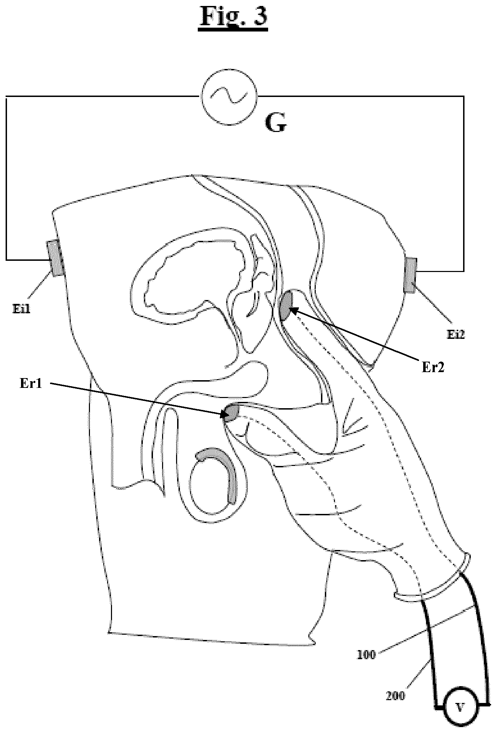

Therefore, figure 3 outlines the generator (G) with the injecting electrodes (Ei1, Ei2), supposing a convenient position of application.

Electrodes may be of the classic type, for example adhesive, is such a manner so as to be easily applicable to the desired points.

In this case, in order to saturate the volume under examination, it may be useful to apply electrodes in the posterior gluteus area and frontally in the bladder area.

Therefore, the generator and the injecting electrodes allow to inject a constant current which saturates the segment under measurement.

The figure shows clearly the gloves with the receiving electrodes Er1 and Er2 allowing to trace back to the potential difference drop, such electrodes being connected to a voltmeter (**V**) through a suitable wiring 100 and 200.

As alternative to the voltmeter, as already mentioned, a device for detecting the phase or both may be foreseen.

The wiring may foresee two cables (100, 200) that are glued along the glove and connected to the electrodes and having the two ends configured for being connected to a pre-existing voltmeter V (or a device for detecting a phase).

As alternative, gloves with electrodes suitable for connecting themselves with a pre-existing wiring and related external voltmeter V (or device for detecting the phase).

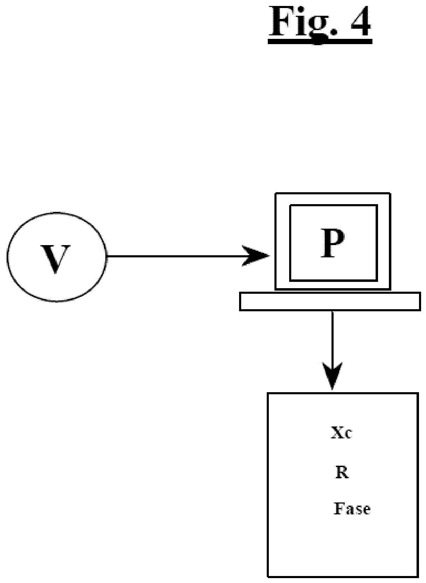

As highlighted further in figure 4, the measured potential difference value, following a known injected current, allows to trace back to various bioelectrical impedance values, such as resistance, reactance and phase, with well-known formulas. From this point, it is possible to trace back rapidly to normality or anomaly conditions of the prostate during the examination, as it is explained further.

Provided the description above, a particularly simple preferred embodiment of the invention foresees a glove having only one electrode.

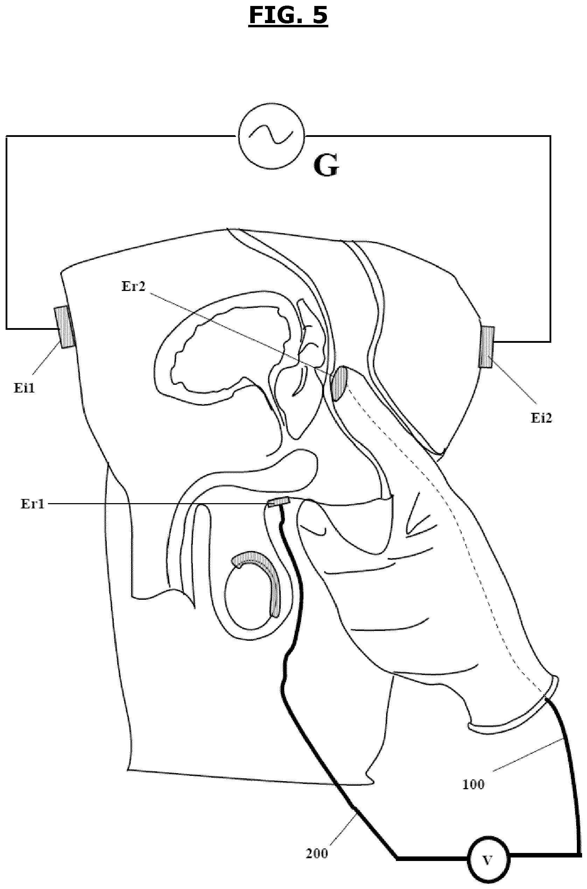

In this case, as shown in figure 5, one of the two receiving electrodes (for example, the electrode Er2 is shown in figure) is an external element and separated from the glove and connecting itself through its own wiring 200 with the voltmeter or, equally, with the phase system. Therefore, in that case, the glove foresees the sole electrode Er2, which connects itself with the voltmeter (or device for detecting the phase).

This solution has, obviously, the advantage of resulting constructively simple, without invalidating the measurement precision, thus allowing to apply other electrodes on preferred points. In fact, the technical problem is reaching the prostatic area with one electrode. Therefore, in that sense, it is necessary to integrate at least an electrode into the glove. The remaining three electrodes are placed on external parts of the body and they are easily reachable: for this reason, normally sold well-known auto-adhesive external electrodes may be used.

Moreover, this allows to vary the position of the third electrode freely, thus adjusting the perception zone of the current saturation area and making the sanitary operator's operation easier, as he will only be involved in palpating the anal canal correctly without maintaining the thumb in contact with the external part of the rectum at the same time.

For example, the preferred arrangement of the invention and therefore the preferred implementation method foresees:
Both an injecting posterior perianal electrode and an injecting electrode placed on the penis, being connected with the generator; both an injecting receiving electrode placed on the glove and a further injecting receiving electrode separated from the glove and placed at the bottom of the penis, being connected with the voltmeter and/or systems for detecting the phase.

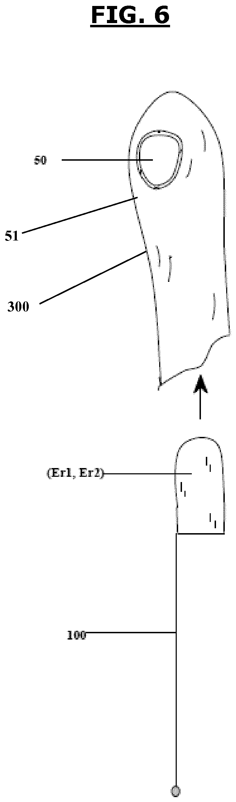

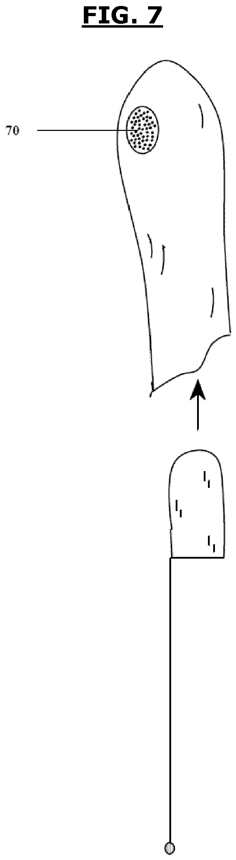

Figure 6 and figure 7 show executive details of the invention and obviously valid for each described configuration.

Figure 6 shows the part 300 of the glove tracing the form of a finger and such a part is provided with a hole 50. For example, the hole can be strengthened with a cuff 51 of material (also the same material of the glove) defining the hole itself, thus strengthening it along its perimeter in order to avoid accidental rips.

The receiving conductive element (Er1, Er2), that is in detail the above-described receiving electrode/s, can be made with such a shape to trace the form of the finger apex, in such a manner that a user can apply it on top of the finger, exactly like a thimble, in quick, easy and stable way.

It is preferably made of AISI 316 steel, which is considered to be biocompatible and therefore does not require any approval test.

Nevertheless, any conductive material (metallic or non-metallic ones) may be used.

For example, aluminum would result particularly suitable, as it is easily malleable, light and cheap. It can be processed easily in such a manner so as to obtain a thin layer. It could be easily created a unique thimble body (Er) with an incorporated conductive fiber 100 made of aluminum.

Provided that, the receiving thimble-shaped electrode is therefore inserted into the glove in such a manner so as to bring it in correspondence of the finger of the glove wherein the hole 50 is obtained. Therefore, the hole creates an opening allowing the metallic element 60 (and therefore the electrode) to be in direct contact with the skin without the interposition of the glove material, which would work as insulation.

The injecting electrode can be both disposable and sterilizable and therefore, in such a case re-applicable on other gloves. The glove is generally disposable.

In this way, the glove with the hole and the electrode are produced and sold separately.

Only gloves arranged to receive receiving electrode/s (Er1, Er2) can be produced and sold separately, as described, and only receiving electrodes can be produced and sold to be used on such pre-pierced gloves, as described.

The realization of assemblies foreseeing a glove with one or more integrated, potentially disposable, receiving electrodes might be taken into account.

Integrated electrodes are arranged in such a manner so as be exposed towards the outside of the glove, for having a conductive direct contact with the skin of the subject.

Such a solution with the electrode in the form of a thimble is efficient electrically, as the resistance of the receiving electrode is further optimized by the resistance of the finger inserted into it, thus improving its functionality (like two parallel resistances **R1** and **R2**).

Provided that, a further variation is shown in figure 1, wherein a plurality of micro-holes 70 replaces the single hole 50.

Such a solution has the advantage of better resistance of the glove against rips. Nevertheless, the use of a conductive gel for better connecting the outside with the electrode and therefore to improve conductivity.

In use, the glove allows to detect electrical bioelectrical impedance parameters allowing the classification of the prostate and a potential medical interpretation of such measurements.

In fact, each healthy subject has got bioelectrical impedance values that remain within a certain range. Subject affected by determined diseases may deviate from such values, as tissues and therefore their conductivity may be altered physiologically.

Therefore, by taking measurements on specimens, diagrams of such bioelectrical impedance parameters can be created easily, thus defining normality and abnormality areas.

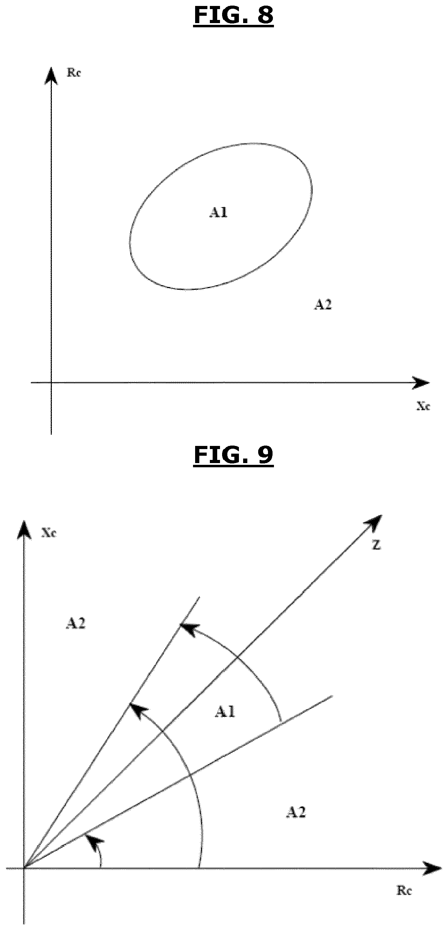

For such a purpose, figure 8 and figure 9 show two diagrams: one of them plots resistance value (Rc) towards electrical reactance (Xc), while the other diagram shows the phase (in the case of a device for detecting a phase variation).

The phase values (ϕ), as well as resistance and reactance, as mentioned above, vary in function of the condition of the organ (therefore, in function of its shape, inflammatory conditions, etc.), as a predetermined pathological condition alters undoubtedly the conductive condition.

Such a principle is therefore used only once to create such diagrams, by analyzing a sample of people, thus creating A1 normality areas and A2 abnormality areas.

At this point, in use, during the check, it is possible to put on the above-mentioned glove connected with the voltmeter (V) and position suitably the injecting electrodes to inject current. The doctor can introduce he finger into the anal orifice and make a test by bringing the corresponding electrode in contact with various points of the prostatic area. Automatically, the acquired bioelectrical impedance parameters, point by point, are plotted to the display into one or both diagrams, thus verifying immediately and undoubtedly whether they remain within a normality range or not.

The measured values plotted on the diagram may then be used for the various medical interpretations of the case.

In the case of a disposable glove with receiving electrodes of the sterilizable type, or in any case separated from the glove, the operator inserts the electrode in the form of a thimble on the corresponding finger and then puts on the glove, in such a manner that the hole corresponds with the part of the thimble. If the configuration with only one receiving electrode inserting on the glove is used, then the operator will have to arrange not only injecting electrodes but also the other receiving electrode on the subject.

If two receiving electrodes in the form of a thimble are foreseen, then both are worn and then the glove is worn.

In the case of the glove having already integrated electrodes, then it is enough to put on the glove normally for starting the test.

Finally, it is highlighted how such a described glove is useful to determine various diseases and conditions of prostatic anomaly, even potential neoplasia.

In fact, it is itself known, from Jossinet's publications, that the analysis of the phase allowed to distinguish an adenoma from carcinoma (benign or malignant tumor).

It is believed that, by transferring such a knowledge to the field of prostate analysis, the present invention allows the obtainment of such prognoses concerning the prostate, as a strong phase variation may be linked to a potential condition of malignant tumorous.

The use of a covering of a sole finger with sanitary material, such as those of the gloves, for example elastic, and on which the electrode is foreseen, is considered an obvious equivalent for its good adherence to the finger and to avoid its slipping in use.

The same can be applied to two coverings of two separated fingers and provided with electrodes.

## Claims

1. A glove for sanitary use (3) for the check of the prostate (20) **characterized by** comprising only one receiving electrode (**Er1, Er2**) arranged in correspondence of a finger of the glove, said receiving electrode resulting at least in part not covered by the material of the glove and thus resulting in direct contact with the outside.

2. A glove for sanitary use (3) for the check of the prostate (20) **characterized by** comprising only two receiving electrodes (**Er1, Er2**) arranged one in correspondence of a finger of the glove and the other one in correspondence of another finger of the glove, different from the previous one, said two receiving electrodes resulting at least in part not covered by the material of the glove and thus resulting in direct contact with the outside.

3. A glove (3), as per claim 1 or 2, wherein such a receiving electrode or said two receiving electrodes are in direct contact with the outside trough:
- A single hole (50) obtained in the application area of the electrode;
- A plurality of micro-holes (70) obtained in the application area of the electrode;

4. A glove (3), as per one or more of the previous claims, wherein the single electrode or the two electrodes are arranged at the apex or near the apex of the finger where they are applied.

5. A glove (3), as per one or more of the previous claims, wherein in the case of only one electrode, this one is arranged in correspondence of the index finger and in the case of two electrodes, one results arranged in correspondence of the index finger and the other one in correspondence of the thumb.

6. A glove (3), as per one or more of the previous claims, wherein said single electrode/s or two electrodes are shaped in the form of thimble.

7. A glove (3), as per one or more of the previous claims, wherein, in the case of only one electrode, a wiring is comprised (100, 200) and arranged inside the glove and connected to the electrode at one side and having the opposite end configured for being connected with a voltmeter or a device for detecting a phase variation and in the case of two electrodes, two wirings are foreseen arranged inside the glove, each one connected with an end to an electrode and with the opposite configured for being connected to a voltmeter (V) or a device for detecting a phase variation.

8. A glove (3), as per claim 7, wherein the wiring/s are glued inside the glove.

9. A glove (3), as per one or more of the previous claims from 1 to 6, wherein said single or two injecting electrodes are configured for being connected with a voltmeter and/or a device for detecting a phase variation.

10. An assembly (1) for the check of the prostate in accordance with the tetra-polar technique and comprising:
- A glove for sanitary use, as per one or more of the previous claims;
- A voltmeter or a device for detecting a phase variation;
- A couple of injecting electrodes;
- A generator (G).

11. An assembly (1), as per claim 10, wherein a processor (P) is comprised suitable for elaborating bioelectrical impedance data.

12. The combination of:
- A glove for sanitary use having a hole in correspondence of one single finger and one single receiving electrode separated from the glove in correspondence of the single hole in such a manner so as to be exposed at least in part towards the outside, or as an alternative;
- A glove for sanitary use having a hole in correspondence of two fingers only for each one of the two fingers and two receiving electrodes separated from said glove and both configured to be inserted each one in correspondence of the relative finger where is the hole in such a manner so as to expose both towards the outside directly.

13. The combination, as per claim 12, wherein the either the glove and/or the receiving electrodes are of the disposable type.
